# EUROPEAN PATENT APPLICATION

(11) **EP 1 772 123 A1**
(43) Date of publication of application: **11.04.2007**
(21) Application number: 06122000.0
(22) Date of filing: 09.10.2006
(51) Int. Cl.: A61F 5/01, A43B 7/26

(54) **Shoe, in particular for prevention and correction of Hallux Valgus**

(30) Priority: 07.10.2005 IT TO20050715
(71) Applicant: Orthofoot S.r.l., 37100 Verona (IT)
(72) Inventor: Boran, Dario, 37126 Verona (IT); Montanari, Michele, 37014 Castelnuovo del Garda (IT); Angi, Giovanni, 35133 Padova (IT)
(74) Representative: Jorio, Paolo

(57) **Abstract**

A shoe (1), for correction and prevention of hallux valgus, has a sole (2); a traction device (11) designed to maintain lateral pull on the hallux (10); and a rigid portion (5) located along one edge (6) of the sole (2) to support an inner lateral portion (8) of the foot, so as to define a fulcrum by which to exert leverage on the hallux (10) by means of the traction device (11).

## Description

The present invention relates to a shoe for prevention, correction, and post-operative treatment of all primary and/or secondary deficiency and instability syndromes of the first ray of the foot, and in particular of hallux valgus.

Various devices are currently marketed for conservative treatment of the above pathology:
- passive or semiactive braces (with elastic traction) worn when the foot is at rest (e.g. at night);
- active braces worn when walking, with a mechanical metatarsophalangeal joint;
- retractors of various shapes and materials, inserted between the first and second toe.

The above devices have been found to be clinically ineffective and/or to cause considerable discomfort to patients. The so-called passive systems, in particular, are relatively ineffective, while the wedge-shaped appliances inserted between the toes tend to deform the other toes if worn when walking.

It is an object of the present invention to provide a shoe designed to solve the above problems in a straightforward, low-cost manner, and which preferably neutralizes and counteracts undesired biomechanical moments resulting in the pathology of hallux valgus, through instability and deficiency of the first ray, permits constant rehabilitative or preventive exercise when walking, and is highly effective.

According to the present invention, there is provided a shoe, in particular for correction and prevention of hallux valgus, comprising:
- a sole;
- traction means designed to maintain lateral pull on the hallux; and
- a rigid lateral supporting portion located along one edge of the sole and, in use, alongside an inner lateral portion of the foot, to define a fulcrum by which to exert leverage on the hallux by means of said traction means.

The effectiveness of the shoe lies in eliminating torsion along the longitudinal axis of the first ray, which is thus supported stably. Corrective traction of the hallux realigns the flexor-extensor tendon system with the centre of rotation of the hallux articulation, which thus regains pull off force.

The purpose of the shoe is therefore not only to have a passive effect, by means of the toe traction strap, but, above all, an active effect by realigning the dynamic components of the articulation to permit correct deambulation. The dynamic components of a misaligned articulation, in fact, are what gradually cause the deformity.

The shoe is designed not only for prevention but also for correction and rehabilitation of hallux valgus, in the event pathology requiring surgery.

A non-limiting embodiment of the invention will be described by way of example with reference to the accompanying drawings, in which:
Figure 1 shows a schematic plan view of a preferred embodiment of the shoe according to the present invention;
Figure 2 shows an exploded view in perspective of a first variation of the Figure 1 shoe;
Figure 3 shows a second variation of the Figure 1 shoe, with parts removed and with one component part extracted from the rest of the shoe for the sake of clarity.

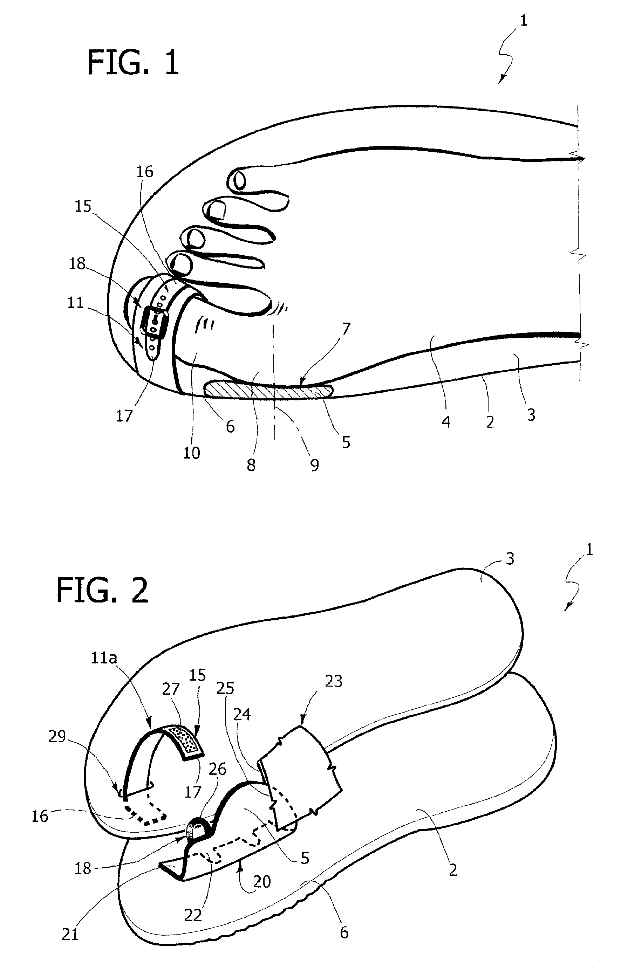

With reference to Figure 1, number 1 indicates as a whole a shoe (shown partly and schematically) comprising a sole 2; and an inner sole 3 superimposed on sole 2, and preferably of the type known by the trade name "fussbett", for supporting the sole of a user's foot 4.

Shoe 1 also comprises a rigid lateral supporting portion 5, which is fixed to sole 2, is located along an inner edge 6 of sole 2, extends upwards with respect to inner sole 3, and has a surface 7 substantially parallel to the longitudinal axis of foot 4 and supporting an inner lateral portion 8 of the forefoot defined, in particular, by the head of the first ray of the first metatarsus, or so-called "bunion".

Portion 5 is made of relatively light material, e.g. nylon or glass and carbon fibre, and is of such a thickness, e.g. 2-3 millimetres, as to be rigid enough, in a direction 9 perpendicular to surface 7, to define, in use, a fulcrum by which to exert leverage on the hallux 10 by means of a traction device 11 in front of portion 5.

More specifically, in view of the fact that, in a hallux valgus condition, the metatarsophalangeal articulation has a bend angle with respect to the normal condition, device 11 is designed to maintain lateral pull on the hallux 10, i.e. parallel to direction 9 and away from the other toes of foot 4. The combined action of the stiffness of portion 5 and traction by device 11 must preferably be such as to reduce the bend angle by at least 50%.

More specifically, device 11 comprises a strap 15, which has a first end 16 fixed directly or indirectly to sole 2, extends about hallux 10, and has a second end 17 secured releasably by a fastening system 18, e.g. a buckle, which is adjustable to adjust the fastening point of end 17 and hence the traction exerted on hallux 10.

In the case of post-operative shoes, sole 2 is preferably more rigid than those of normally marketed shoes, to minimize normal toe movement when walking.

Figure 2 shows a variation of shoe 1, in which the component parts are indicated, where possible, using the same reference numbers as in Figure 1. Portion 5 forms part of a rigid L-shaped body 20 comprising an indented base wall 21 crosswise to portion 5, fixed to sole 2, and interposed between sole 2 and inner sole 3. Body 20 also comprises a portion 22 defining a front extension of portion 5 long enough, e.g. 5-6 centimetres, to extend alongside the whole of hallux 10.

Shoe 1 also comprises a vamp 23 (shown partly) in turn comprising an inner layer 24 or "liner", and an outer layer or "vamp" proper, which are stitched together, and between which is interposed the plate defined by portions 5 and 22.

The rear (not shown) of vamp 23 preferably defines a lateral support on both sides of the heel of the foot, and so clamps the heel laterally. In other words, the rear of vamp 23 is designed to prevent lateral movement of foot 4 when walking (rear-foot fulcrum), and so provides additional support, in addition to portion 5, for leverage by device 11. The shoe therefore provides for gradually rebuilding the medial longitudinal arch lost as the hallux valgus condition deteriorates.

Portion 22 supports a fastening or transmission ring 26, which extends outside vamp 23 and forms part of a traction device 11a similar to device 11. Device 11a differs from device 11 by system 18 comprising a strip of Velcro 27 on strap 15, as opposed to a buckle. More specifically, strap 15 extends through a slit 29 in inner sole 3, and end 17 is threaded through ring 26 and folded back on itself to fix Velcro 27 to an intermediate portion of strap 15.

Ring 26 is inserted between vamp 25 and liner 24 and fixed to a preferably 8 mm wide nylon tape (not shown); and the end of the tape is then stitched to secure it to vamp 25 and liner 24.

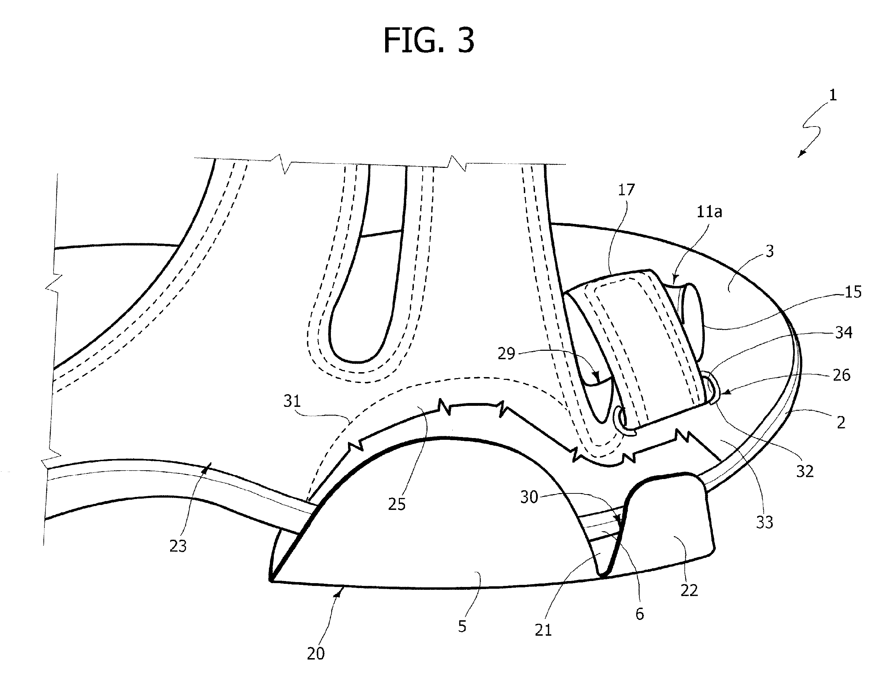

Figure 3 shows a variation of shoe 1, in which the component parts are indicated, where possible, using the same reference numbers as in Figure 2. Unlike the Figure 2 solution, the vertical plate defined by portions 5 and 22 has a substantially vertical, V-shaped slit 30 separating portions 22 and 5. Slit 30 preferably extends down to wall 21, and allows wall 21 of body 20 to bend to move portions 22 and 5 towards and away from each other when walking. Layers 24, 25 of vamp 23 are joined by stitching 31 above the top edge of the vertical plate, and which therefore partly follows the outline of slit 30.

Moreover, portion 22 supports ring 26 indirectly : ring 26 comprises a bottom fastening portion 32 connected to a portion 33 of layer 25 covering portion 22, so that ring 26 rests on portion 22 with the interposition of portion 33. Ring 26 also comprises a top portion 34, which projects from vamp 23, facing layer 25, to fold back end 17.

As will be clear from the foregoing description, by virtue of portion 5 and device 11, shoe 1 provides for actively preventing or correcting deviation of hallux 10 by realigning the dynamic components of the articulation to permit correct deambulation, and by also preventing any consequences of the hallux valgus condition caused by mechanical deficiency of the first ray (metatarsalgie of other toes, postural alterations such as genu valgum, lumbosacral contractures, etc.).

Shoe 1 provides, in some cases, for avoiding or delaying corrective surgery, and for effectively handling the post-operative period, should surgery be inevitable.

Body 20 is designed to fit easily to sole 2, without affecting the comfort of foot 4 when walking, or the rigidity of the support defined by portion 5.

The fact that part of device 11, in particular ring 26, is fitted to body 20, i.e. connected directly or indirectly to portion 22 of body 20, increases the rigidity of the fulcrum and hence leverage of hallux 10.

Clearly, changes may be made to shoe 1 as described herein without, however, departing from the scope of the present invention as defined in the accompanying Claims.

In particular, shoe 1 may, at least partly, have no vamp, and may be in the form of a sandal, slipper, etc.

Strap 15 may differ in shape and size from those shown, and end 17 may be fixed directly to portion 22, as opposed to being folded back about ring 26.

Ring 26 may be replaced with a different type of fastening or retaining member.

## Claims

1. A shoe (1), in particular for prevention and correction of hallux valgus, comprising:
- a sole (2);
- traction means (11) designed to maintain lateral pull on the hallux (10); and
- a rigid lateral supporting portion (5) located along one edge (6) of the sole (2) and, in use, alongside an inner lateral portion (8) of the foot (4), to define a fulcrum by which to exert leverage on the hallux (10) by means of said traction means (11).

2. A shoe as claimed in Claim 1, **characterized in that** said rigid lateral supporting portion (5) has a supporting surface (7) for the head of the first ray of the first metatarsus.

3. A shoe as claimed in Claim 1, **characterized in that** said rigid lateral supporting portion (5) is fixed to said sole (2).

4. A shoe as claimed in Claim 3, **characterized in that** said rigid lateral supporting portion (5) forms part of a rigid L-shaped body (20) comprising a base wall (21) crosswise to said rigid lateral supporting portion (5), fixed to said sole (2), and interposed between said sole (2) and an inner sole (3) superimposed on said sole (2).

5. A shoe as claimed in any one of the foregoing Claims, **characterized by** also comprising a vamp (23) having an inner layer and an outer layer (24, 25); said rigid lateral supporting portion (5) being interposed between said inner and said outer layer (24, 25).

6. A shoe as claimed in any one of the foregoing Claims, **characterized in that** said rigid lateral supporting portion (5) forms part of a substantially vertical lateral plate, which in turn forms part of a one-piece body (20) and comprises a supporting portion (22) in front of said rigid lateral supporting portion (5); at least part of said traction means (26) being supported by said supporting portion (22).

7. A shoe as claimed in Claim 6, **characterized in that** said traction means (11) comprise:
- a member (26) fitted directly or indirectly to said supporting portion (22);
- a strap (15), which has a first end (16) fixed with respect to said sole (2), able to extend about the hallux (10), and is connectable to said member (26);
- fastening means for securing a second end (17) of said strap (15).

8. A shoe as claimed in Claim 7, **characterized in that** the fastening point of said second end (17) is adjustable to adjust the pull exerted on said hallux (10).

9. A shoe as claimed in Claim 8, **characterized in that** said fastening means (16) comprise Velcro (27) on a surface of said strap (15).

10. A shoe as claimed in any one of Claims 7 to 9, **characterized in that** said member (26) is defined by a ring, through which said second end (17) is threaded before being fastened.

11. A shoe as claimed in any one of Claims 6 to 10, **characterized in that** said substantially vertical lateral plate has a slit (30) separating said supporting portion (22) and said rigid lateral supporting portion (5).
